## Europäisches Patentamt

## European Patent Office

## Office européen des brevets

(11) Veröffentlichungsnummer: **0 420 075 B1**

# EUROPÄISCHE PATENTSCHRIFT

(45) Veröffentlichungstag der Patentschrift: **24.05.95**

(51) Int. Cl.6: **C12Q 1/40**

(21) Anmeldenummer: **90118228.7**

(22) Anmeldetag: **22.09.90**

(54) **Verfahren zum Bestimmen des Anteils eines Fusionsproteins in einem Gemisch aus Fusionsprotein und proteolytischem Abbauprodukt.**

(30) Priorität: **27.09.89 DE 3932180**

(43) Veröffentlichungstag der Anmeldung:
**03.04.91 Patentblatt 91/14**

(45) Bekanntmachung des Hinweises auf die Patenterteilung:
**24.05.95 Patentblatt 95/21**

(84) Benannte Vertragsstaaten:
**BE CH DE DK FR GB LI NL SE**

(56) Entgegenhaltungen:

**BIO/TECHNOLOGY, Band 7, Nr. 10, Oktober 1989, New York, NY (US); K.-P. KOLLER et al., Seiten 1055-1059&NUM;**

**EUROPEAN JOURNAL OF BIOCHEMISTRY, Band 141, Nr. 3, März 1984, Berlin (DE); L. VERTESY et al., Seiten 505-512&NUM;**

**FEMS MICROBIOLOGY LETTERS, Band 65, Juli 1989, Amsterdam (NL); W. ARETZ et al., Seiten 31-36&NUM;**

**DIABETOLOGICA, Band 8, 1972; L.H. HEDING, pp. 260-266&NUM;**

(73) Patentinhaber: **HOECHST AKTIENGESELL-SCHAFT**

**D-65926 Frankturt (DE)**

(72) Erfinder: **Bender, Rudolf, Dr.**
**Oranienstrasse 5**
**D-6232 Bad Soden (DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Bei der Fermentation rekombinanter Streptomyceten zum Herstellen eines Fusionsproteins mit Tendamistat-Anteil wird ein proteolytischer Abbau des sekretierten Proteins durch Proteasen festgestellt, die neben dem Fusionsprotein bei der Fermentation entstehen. Durch die Einwirkung der Proteasen bleibt als Bestandteil des Fusionsproteins im wesentlichen das Tendamistat als Abbauprodukt erhalten. Da bekannt ist, daß Tendamistat ein Peptid-Inhibitor der Pankreas α-Amylase ist, kann die Gesamtproduktbildung einer Streptomycetenkultur, also der Gehalt an Fusionsprotein einschließlich Abbauprodukt, mit dem α-Amylase-Inhibitor-Test ermittelt werden. Nachteilig ist jedoch der Zeitaufwand zur Ermittlung des Gehalts an reinem Fusionsprotein, der für den Aberntezeitraum oder bei der Produktisolierung von Bedeutung ist. So dauert die Bestimmung des Fusionsproteins nach der Sodiumdodecylsulfat (SDS)-Elektrophorese mindestens 8 Stunden und nach der Insulin-Radioimmunoassay (RIA)-Methode ca. 24 Stunden. Hier will die Erfindung Abhilfe schaffen.

Es wird ein Verfahren zum Bestimmen des Anteils von Fusionsprotein in einem Gemisch aus Fusionsprotein mit Tendamistat-Anteil und proteolytischem Abbauprodukt vorgeschlagen, daß dadurch gekennzeichnet ist, daß man das Gemisch aus Fusionsprotein mit Tendamistat-Anteil und Abbauprodukt mit Rinderserumalbumin verdünnt, einen Teil des verdünnten Gemisches mit α-Amylase-Lösung mischt, ≧30 Min. bei 37° C inkubiert, α-Amylase-Reagenz zuführt und die Restaktivität der α-Amylase-Lösung spektrophotometrisch mißt, den anderen Teil des verdünnten Gemisches mit α-Amylase-Reagenz mischt, nach dem Mischen α-Amylase-Lösung zusetzt und die Enzymkinetik (Reaktionsverlauf) spektrophotometrisch mißt, alle Meßwerte mit Standards aus Fusionsprotein, Tendamistat Abbauprodukt und ungehemmter α-Amylasereaktion vergleicht und aus dem Vergleich den Gehalt an Fusionsprotein im Gemisch bestimmt.

Bei dieser Methode, nach der sich der Gehalt an intaktem Fusionsprotein im Gemisch mit Abbauprodukt in weniger als 2 Stunden mit einer durchschnittlichen Fehlerbreite von ±10 % bestimmen läßt, nutzt man also die unterschiedlichen Ratenkonstanten bei der Reaktion von gereinigtem Fusionsprotein, Abbauprodukt und Tendamistat mit α-Amylase.

Im folgenden wird die Erfindung anhand eines Beispiels näher erläutert:

Die Fermenterbrühe wird zentrifugiert oder filtriert, um den Mikroorganismus sowie unlösliche Mediumsbestandteile abzutrennen. Der Zentrifugenüberstand oder das Filtrat entspricht dem oben definierten Gemisch aus Fusionsprotein und proteolytischem Abbauprodukt. Dieses Gemisch wirdsofern sein pH-Wert ±1,5 von 7 abweicht - mit HCl oder NaOH neutralisiert und dann mit 0,1 %iger Rinderserumalbumin-Lösung im Verhältnis 1:100 verdünnt und das verdünnte Gemisch in einem Zweischrittverfahren auf α-Amylase-Hemmung getestet.

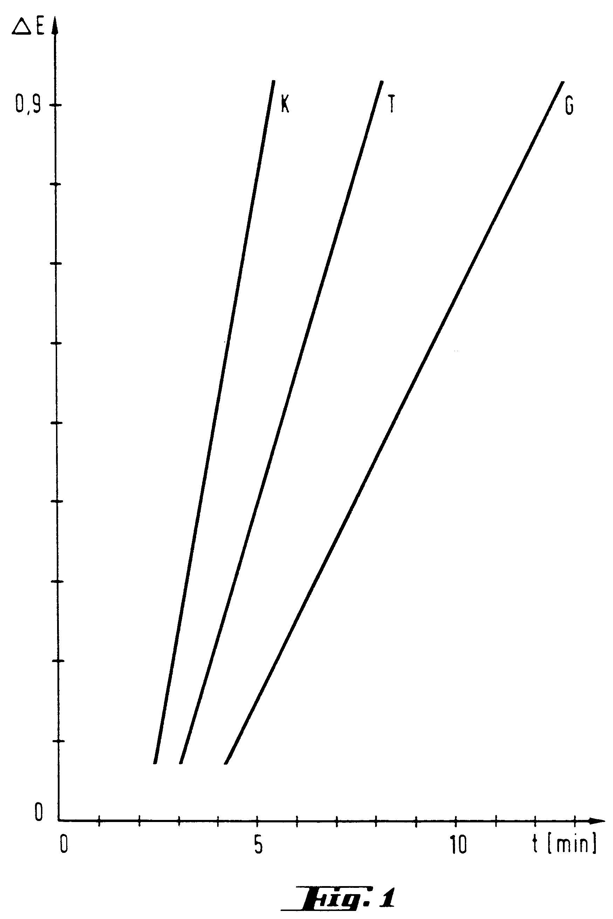

Im ersten Schritt werden 0,1 ml des verdünnten Gemisches mit 0,4 ml α-Amylase-Lösung gemischt. Die α-Amylase-Lösung wird hergestellt durch Lösen von 8 mg α-Amylase aus Schweinepankreas der Firma Merck, Darmstadt, Bestell-Nr. 16 312 in 1 l Lösung enthaltend 100 mMol HEPES-Puffer (pH 7), 2 mMol CaCl$_2$ sowie 0,4 % Rinderserumalbumin. Darüberhinaus werden 0,1 ml 0,1 %iger Rinderserumalbumin-Lösung zur Kontrolle der ungehemmten Reaktion sowie 0,1 ml reine Tendamistat-Lösung in der Konzentration 1 mg pro l 0.1 %iger Rinderserumalbumin-Lösung als Inhibitorstandard mit jeweils 0,4 ml α-Amylase-Lösung vermischt. Die drei Reaktionsansätze werden ≧30 Min. bei 37° C inkubiert. Von jedem inkubierten Ansatz werden 20 µl mit 1 ml α-Amylase-Reagenz (WAK Chemie, Bad Soden), das auf 37° C vortemperiert ist, vermischt. Unmittelbar nach dem Mischen wird die Reaktionsrate V, bei der durch das α-Amylase-Reagenz vorgegebenen Wellenlänge von 405 nm ca. 5 Min. photometriert und die Extinktion ΔE in Abhängigkeit von der Zeit (t) registriert. Die Reaktionsrate V ist bei dem verwendeten α-Amylase-Reagenz linear. Sie beträgt gemäß Figur 1 für das Gemisch aus Fusionsprotein und Abbauprodukt 0,10 ΔE pro Minute, für die Kontrollreaktion 0,3 ΔE pro Minute und für die Tendamistat-Reaktion 0,16 ΔE pro Minute. Da die Reaktionsrate V proportional der Inhibitorkonzentration C ist, läßt sich die Inhibitorkonzentration $C_G$ des Gemisches aus Fusionsprotein und Abbauprodukt aus folgendem Gleichungssystem ermitteln.

$$V_G = -mC_G + V_K$$
$$V_T = -mC_T + V_K$$

wobei

$V_G$     die Reaktionsrate des Gemisches (G)

$V_T$     die Reaktionsrate der Tendamistat-Lösung (T)

$V_K$     die Reaktionsrate der Kontroll-Lösung (K)

$C_G$     die Konzentration des Fusionsproteins + Abbauprodukt [mg/l] im Gemisch

$C_T$     die Konzentration von Tendamistat in der Tendamistat-Lösung

Da $C_T = 1$ mg/l gewählt wurde ergibt sich aus dem Gleichungssystem für

$$C_G = \frac{V_K - V_G}{V_K - V_T} = \frac{0,3 - 0,1}{0,3 - 0,16} = 1,43 \text{ mg/l}$$

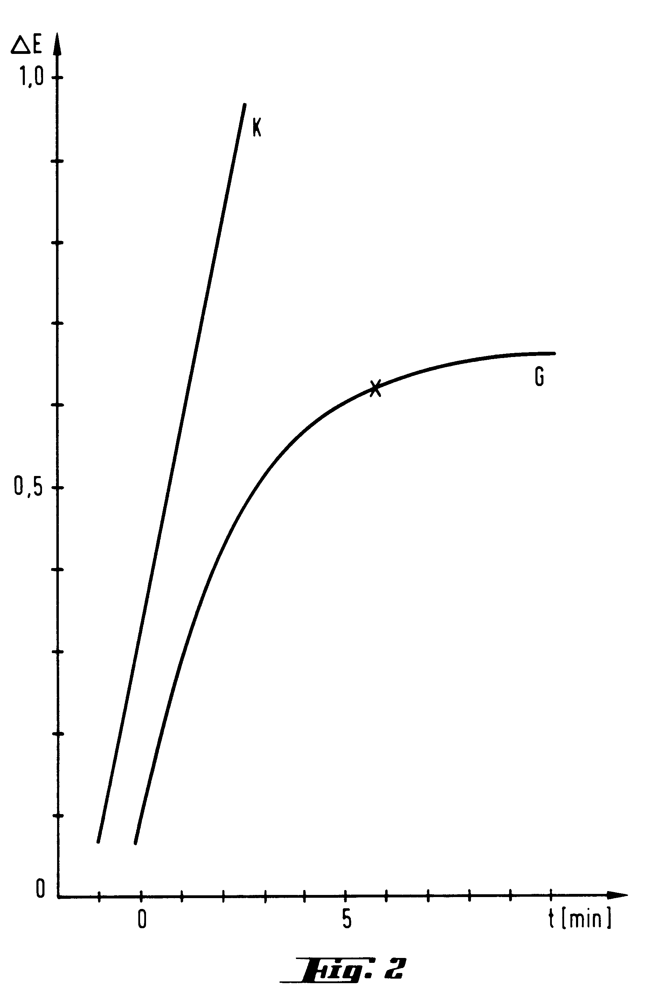

Im zweiten Schritt werden 150 $\mu$l des 1:100 verdünnten Gemisches aus Fusionsprotein und Abbauprodukt mit 800 $\mu$l $\alpha$-Amylase-Reagenz (WAK Chemie, Bad Soden) gemischt und auf 37°C temperiert. Ebenso werden 150 $\mu$l 0,1 %iger Rinderserumalbumin-Lösung zur Kontrolle mit 800 $\mu$l $\alpha$-Amylase-Reagenz gemischt und auf 37°C temperiert. Die Reaktion des Gemisches wird durch Zugabe von 50 $\mu$l $\alpha$-Amylase-Lösung, wie nach Schritt 1 - jedoch mit einer $\alpha$-Amylase-Konzentration von 2,5 mg/l - hergestellt, gestartet und der Reaktionsverlauf (Kinetik) für ca. 10 Minuten bei 405 nm photometriert und die Extinktion ($\Delta$E) in Abhängigkeit von der Zeit registriert (Figur 2). In Gegenwart von Inhibitor nimmt die Reaktionsrate nach dem Start der Reaktion stetig ab. Der Reaktionsverlauf der Rinderserumalbumin-Lösung (Kontrolle) ist linear; die Reaktionsrate $V_K$ beträgt 0,26 $\Delta$E pro Minute bei diesem Ansatz. Figur 2 ist zu entnehmen, daß die Reationsrate $V_G$ für das Gemisch aus Fusionsprotein und Abbauprodukt zwischen der 5. und 6. Minute 0,02 $\Delta$E pro Minute beträgt.

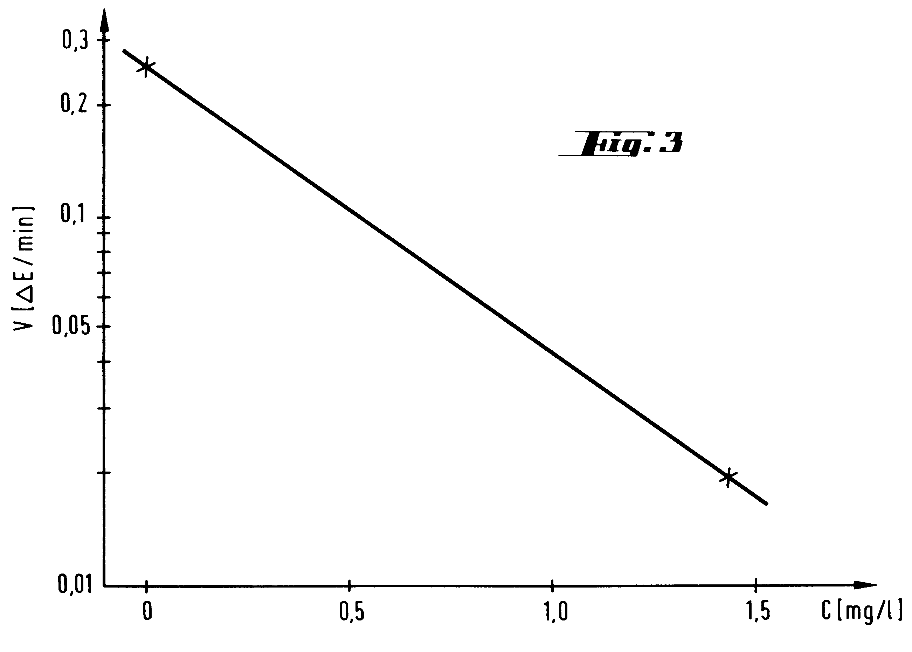

Die aus dem Reaktionsverlauf von Schritt 2 ermittelten Reaktionsraten $V_G$ und $V_K$ werden halblogarithmisch gegen die nach Schritt 1 aus den Reaktionsraten $V_G$ $V_T$ und $V_K$ ermittelte Konzentration $C_G$ des Gemisches aus Fusionsprotein und Abbauprodukt graphisch dargestellt (Figur 3). Es ergibt sich eine Gerade, deren Steigung ein Maß für den Gehalt an Fusionsprotein im Gemisch aus Fusionsprotein und Abbauprodukt darstellt. Verglichen wird dieses Maß mit der Steigung von Standardkurven (Geraden), die nach der gleichen Methode aus reinem Abbauprodukt und reinem Fusionsprotein gewonnen wurden.

**Patentansprüche**

1. Verfahren zum Bestimmen des Anteils eines Fusionsproteins in einem Gemisch aus Fusionsprotein mit Tendamistat-Anteil und proteolytischem Abbauprodukt, dadurch gekennzeichnet, daß man das Gemisch aus Fusionsprotein mit Tendamistat-Anteil und Abbauprodukt mit Rinderserumalbumin verdünnt, einen Teil des verdünnten Gemisches mit $\alpha$-Amylase-Lösungmischt, $\geq$30 Minuten bei 37°C inkubiert, $\alpha$-Amylase-Reagenzzufuhr und die Restaktivität der $\alpha$-Amylase-Lösung spektrophotometrisch mißt, den anderen Teil des verdünnten Gemisches mit $\alpha$-Amylase-Reagenz mischt, nach dem Mischen $\alpha$-Amylase-Lösung zusetzt und die Enzymkinetik (Reaktionsverlauf) spektrophotometrisch mißt, alle Meßwerte mit Standarts aus Fusionsprotein, Tendamistat, Abbauprodukt und ungehemmter $\alpha$-Amylase-Reaktion vergleicht und aus dem Vergleich den Gehalt an Fusionsprotein im Gemisch bestimmt.

**Claims**

1. A process for the determination of the content of a fusion protein in a mixture of fusion protein having a tendamistat content and proteolytic degradation product, which comprises diluting the mixture of fusion protein having a tendamistat content and degradation product with bovine serum albumin, mixing a portion of the diluted mixture with $\alpha$-amylase solution, incubating at 37°C for $\geq$30 minutes, introducing $\alpha$-amylase reagent and measuring the residual activity of the $\alpha$-amylase solution by spectrophotometry, mixing the other portion of the diluted mixture with $\alpha$-amylase reagent, adding $\alpha$-amylase solution after mixing, and measuring the enzyme kinetics (course of reaction) by spectrophotometry, comparing all the measurements with standards composed of fusion protein, tendamistat, degradation product and noninhibited $\alpha$-amylase reaction and, from the comparison, determining the content of fusion protein in the mixture.

**Revendications**

1. Procédé de dosage de la fraction de protéine de fusion dans un mélange constitué par une protéine de fusion dont un élément est le tendamistat et par un produit de dégradation protéolytique, caractérisé en ce que l'on dilue avec de la sérum-albumine bovine le mélange constitué par la protéine de fusion dont un élément est le tendamistat et par le produit de dégradation, on mélange une partie du mélange dilué avec une solution d'$\alpha$-amylase, on le met à incuber pendant $\geq$ 30 minutes à 37°C, on ajoute du réactif d'$\alpha$-amylase et on mesure par spectrophotométrie l'activité résiduelle de la solution d'$\alpha$-amylase, on

mélange l'autre partie du mélange dilué avec du réactif d'$\alpha$-amylase, on ajoute après l'opération de mélange une solution d'$\alpha$-amylase, et on mesure par spectrophotométrie la cinétique enzymatique (avancement de la réaction), on compare toutes les valeurs mesurées avec des références pour la protéine de fusion, le tendamistat, le produit de dégradation et la réaction d'$\alpha$-amylase non inhibée, et on détermine à partir de la comparaison la teneur en protéine de fusion du mélange.

Fig. 1

*Fig. 2*

Fig: 3

EP 0 420 075 B1